# EUROPEAN PATENT SPECIFICATION

(11) **EP 1 965 881 B1**
(45) Date of publication and mention of the grant of the patent: **13.05.2009**
(21) Application number: 06848674.5
(22) Date of filing: 20.12.2006
(51) Int. Cl.: B01D 15/08, C07K 17/00

(54) **SYNTHETIC PEPTIDE LIGANDS ABLE TO BIND OCHRATOXIN A AND USES THEREOF**
SYNTHETISCHE PEPTIDLIGANDEN ZUR BINDUNG VON OCHRATOXIN A UND ANWENDUNGEN DAVON
PEPTIDES SYNTHÉTIQUES POUVANT SE LIER À L'OCHRATOXINE A ET LEURS UTILISATIONS

(30) Priority: 23.12.2005 IT TO20050905
(43) Date of publication of application: 10.09.2008
(73) Proprietor: Universita' Degli Studi di Torino, 10124 Torino (IT)
(72) Inventor: TOZZI, Cinzia, 1-10125 Torino (IT); FERROGLIO, Caterina, 1-10125 Torino (IT); GIRAUDI, Gianfranco, 1-10125 Torino (IT); ANFOSSI, Laura, 1-10125 Torino (IT); BAGGIANI, Claudio, 1-10125 Torino (IT)
(74) Representative: Freyria Fava, Cristina
(86) International application number: PCT/IB2006/003817
(87) International publication number: WO 2007/072212

(56) References cited:
- WO-A-03/103409
- MAIER N M ET AL: "Molecularly imprinted polymer-assisted sample clean-up of ochratoxin A from red wine: merits and limitations" JOURNAL OF CHROMATOGRAPHY B: BIOMEDICAL SCIENCES & APPLICATIONS, ELSEVIER, AMSTERDAM, NL, vol. 804, no. 1, 5 May 2004 (2004-05-05), pages 103-111, XP004501565 ISSN: 1570-0232
- BEJAOUI H.: "Ochratoxin A removal in synthetic and natural grape juices by selected oenological Saccharomyces strains." J. APPLIED MICROBIOL., vol. 97, 2004, pages 1038-1044JJ, XP002453478
- VARGA J.: "Recent advances in ochratxin research : production, detection and occurrence of ochratoxins." CEREAL RESEARCH COMMUNICATION, vol. 29, no. 1-2, 2001, pages 85-92, XP009090275
- DATABASE WPI Week 2005 Derwent Publications Ltd., London, GB; AN 2005-754211 XP002453482 & JP 2005 306762 A (UNIV. NAGOYA) 4 November 2005 (2005-11-04)

### SEQUENCE LISTING

<110> università degli studi di Torino
<120> Synthetic ligands able to bind ochratoxin A and uses thereof
<130> BWO8306-CF
<140> PCT/IB2006/003817
   <141> 2006-12-20
<150> TO2005A000905
   <151> 2005-12-23
<160> 9
<170> Patent In version 3.3
<210> 1
   <211> 6
   <212> PRT
   <213> artificial
<220>
   <223> peptide specific for ochratoxin A
<400> 1
   Ser Asn Leu His Pro Lys
   1               5
<210> 2
   <211> 6
   <212> PRT
   <213> artificial
<220>
   <223> peptide specific for ochratoxin A
<400> 2
   Ser Asn Leu His Asn Ser
   1               5
<210> 3
   <211> 6
   <212> PRT
   <213> artificial
<220>
   <223> peptide specific for ochratoxin A
<400> 3
   Ser Asn Leu His Phe Gly
   1 5
<210> 4
   <211> 6
   <212> PRT
   <213> artificial
<220>
   <223> peptide specific for ochratoxin A
<400> 4
   Ser Asn Leu His Trp Gly
   1 5
<210> 5
   <211> 6
   <212> PRT
   <213> artificial
<220>
   <223> peptide specific for ochratoxin A
<400> 5
   Ser Asn Leu His Asn Gly
   1 5
<210> 6
   <211> 6
   <212> PRT
   <213> artificial
<220>
   <223> peptide specific for ochratoxin A
<400> 6
   Ser Asn Leu His Leu Lys
   1 5
<210> 7
   <211> 6
   <212> PRT
   <213> artificial
<220>
   <223> peptide specific for ochratoxin A
<400> 7
   Ser Asn Leu His Trp Ser
   1 5
<210> 8
   <211> 6
   <212> PRT
   <213> artificial
<220>
   <223> peptide specific for ochratoxin A
<400> 8
   Ser Asn Leu His Leu Ser
   1 5
<210> 9
   <211> 4
   <212> PRT
   <213> artificial
<220>
   <223> peptide specific for ochratoxin A
<400> 9
   Ser Asn Leu His
   1

## Description

The present invention relates to synthetic ligands, in particular peptides, able to bind with the mycotoxin ochratoxin A and the uses thereof.

The term mycotoxin identifies a series of products toxic to humans and animals which are generated by some fungal species which can contaminate a high number of foods, such as for example cereals, mainly wheat, corn, rye and barley, the oleaginous plants, the coffee, the cacao, the spices, the milk and its derivatives. The infection, the development and the production of mycotoxins can occur during each stage of the productive cycle, from the field cultivation to the harvesting, the storage, the transformation and the transport of the end products. These compounds further result to be chemically very stable and resistant to the processings to which they are subjected. Their frequent incidence has a potential negative impact on the economy of the manufacturing regions, in particular those developing zones where, often, the agricultural techniques are poorly developed. Moreover, there are even serious contamination cases in the Countries with more modern and advanced agricultural systems.

With these premises, the controls for the identification and the quantification [1] of the mycotoxins throughout the agricultural and food chain gain a great importance. The analysis methods are various and exploit different analytical techniques, but generally a protocol for the determination of the mycotoxins is divided in four stages: the sampling, the extraction, the cleaning from the matrix and a possible pre-concentration of the toxin and finally the real quantitative analysis.

A great deal of the commercially analysis methods makes use of antibodies both in the cleaning and preconcentration step, through the use of resins by immunoaffinity, and the analytical step with the use of immunochemical assays. Actually, the antibodies offer a good analytical sensitivity and high affinities, for this reason they are usually very effective in the capturing and the identification of the analytes. In the light of these merits, the use of the antibodies also shows some drawbacks: for example, in case of the stationary phases by immunoaffinity, the costs are rather high due to the poor re-use possibility, the regeneration difficult and the large quantities of antibodies required for their preparation. These problems are essentially due to the deterioration of the bonding ability of the antibodies, which do not tolerate extreme working environments, such as the extracts of the matrixes containing the mycotoxins.

The object of the present invention is to provide synthetic ligands able to bind to the mycotoxin ochratoxin A, where such ligands mime the behavior of the antibodies and have such molecular recognition properties that are used in the analysis methods of a sample (generally a food matrix).

A further object of the present invention is to provide a process for the detection of the ochratoxin A in a sample, preferably a food matrix, through the use of such ligands.

According to the present invention, such objects are attained thanks to the solution stated in a specific way in the following claims. The claims form an integral part of the technical teaching provided herein with reference to the invention.

The invention concerns some peptides and the corresponding binding systems able to selectively bind the mycotoxin ochratoxin A and the uses thereof.

The invention further concerns analysis methods of the ochratoxin A in a sample and solid phases to be used in such analysis methods.

In the ambit of the present invention, by the term "binding system" is understood to mean a molecular system including a molecule/peptide specific for the analyte. The binding system according to the present invention can further include a spacer arm, wherein with such term is understood to mean a natural or synthetic molecular structure directly or indirectly bound to the molecule/peptide specific for the analyte, which allows an easier interaction of the molecule/peptide with the analyte.

### DETAILED DESCRIPTION

The invention will be now described in detail with reference to a presently preferred embodiment, by mere way of not limitative example, with reference to the enclosed figures, in which:
- figure 1 is a graph which represents the bonding test of the dipeptides library towards the ochratoxin A (OA);
- figure 2 is a graph which represents the bonding test of the tetrapeptides library towards the OA at a pH 4;
- figure 3 is a graph which represents the bonding test of the tetrapeptides library towards the OA at a pH 8;
- figure 4 is a graph which represents the bonding test of the hexapeptides library towards the OA at a pH 4;
- figure 5 is a graph which represents the bonding test of the hexapeptides library towards the OA at a pH 8;
- figure 6 is a graph which represents the B/F values of the hexapeptide of SEQ ID NO.: 1 towards the OA at different pHs;
- figure 7 is a graph which represents the B/F values of the hexapeptide of SEQ ID NO.:1 towards the OA at different ionic strengths;
- figure 8 is a graph which represents the result of analysis at different ionic strengths in HPLC;
- figure 9 is a graph which represents the result of the selectivity analysis of the hexapeptide of SEQ ID NO.:1 towards various mycotoxins;
- figure 10 is a graph which represents a chromatogram in which it can be noted the almost total displacement of the peak of the retention time of the ochratoxin A from about 3.8 minutes to 8.5 minutes.

In the last years, the quality control on the foods is become an interesting subject for the scientific community, and for this reason it has been tried to develop analysis methods directed to different analytes: the fundamental requirements of such analysis methods should obviously be the simplicity and the possibility of analyzing a great assortment of matrixes with different characteristics.

The techniques now available are sometimes complicated, expensive and specific for particular matrixes.

The present invention is aimed at the identification of synthetic ligands, more specifically peptides, developed by a combinatorial way for the recognition of mycotoxins existing in determined food matrixes.

With the method above mentioned, some peptides specific for the ochratoxin A have been obtained, whose sequences are defined in SEQ ID NO.: 1-9 able to recognize, to bind and to retain such analyte, thus allowing to separate the molecule of interest from the possible interfering matters, such as the anthocyanins in the wines, normally existing in the real matrixes.

In order to use such peptide for the solid phase extraction of real samples, optimization tests of the bond (for example analyses at different pHs and ionic strengths) have been carried out, for the purpose of optimizing the experimental procedure in order to obtain the maximum recovery and the maximum concentration of ochratoxin A following to the step of solid phase extraction (SPE), for the purpose of recovering the analyte, by eliminating the interferences existing in the solution, in order to subsequently carry out a quantitative evaluation of its HPLC concentration with a fluorescence detection.

The tests carried out for the hexapeptide of SEQ ID NO.:1 binding the ochratoxin A on complex real matrixes have proved to be effective: in fact, it has been possible to detect the presence of ochratoxin A in concentrations equal and also lower than those appearing as limits imposed by the European law. Furthermore, the recoveries found (higher than 75%) agree with those existing in literature with procedures using a solid phase extraction via immunoaffinity.

With respect to the traditional methods, which use, for the separation, small immunoaffinity columns equipped with antibodies difficult and expensive to obtain, the method described in the present application results cheaper, since the peptides can be synthesized in a laboratory without using animals or sophisticated technologies (which require the antibodies), the synthesis procedure are generally simple and with cheap reagents, furthermore the time required for selecting a peptide ligand is usually very reduced with respect to the time needed for the adjustment of a biological ligand. Moreover, being synthesized in a laboratory, the sequences can be easily controlled and purified and do not undergo any variations from batch to batch, as it is the case, on the contrary, for the antibodies. The peptide ligands are very resistant, also in extreme conditions, such as high percentages of solvent, temperature or pH variations or in the presence of complex matrixes; they are utilizable many times (the reusability has been tested after 31 SPEs by providing a result comparable with the one provided the first time, with a recovery of 105.2%) without undergoing modifications which decrease the bonding ability thereof, whereas the antibodies are much more weak and sensitive to the prolonged use.

The affinity constants of the peptides and in particular of the peptide of SEQ ID NO.: 1 subject of the present invention allow to detect quantities of analytes well below the existing limits imposed by the law and to work with extraction conditions more similar to the nature of the sample, differently from what happens for the antibodies.

### Immobilization of the spacer arm

The synthesis has been carried out in a solid phase and aqueous environment following the procedures described in literature [4]. The solid phase used is a polystyrene resin Amberlite IRC-50 (4% crosslinked) functionalized with surface carboxy groups. Furthermore, thanks to the large dimensions (16-50 mesh) the resin is easy to separate and to handle.

First of all, to the surface carboxy groups of the solid phase a mixture of ethanolamine (90%) and aminobutyric acid (10%) has been conjugated, following the method of the activated N-hydroxysuccinimidic ester. [5] The solid phase carboxy groups have been activated with a solution in dry N,N-dimethylformamide of N-hydroxysuccinimide (NHS) and N,N-dicyclohexylcarbodiimide (DCD) in a quantity equimolar with the surface carboxy groups. Therefore, after a washing step [4], the immobilization of the spacer arm, the aminobutyric acid, is carried out always following the procedures described in literature.

### Creation of the first dipeptides library

Using the solid phase above prepared as a starting base, the first combinatorial library has been prepared (144 combinations) according to the diagram shown in table 1. 2.25 grams of resin with spacer arm have been suspended in 36 ml of hydrogen carbonate buffer (0.15 M NaHCO₃, pH 8.5) and a suspension of 0.25 ml well have been distributed in the wells of two microtitre plates with filter septum, for a total of 144 wells. A first amino acid has been conjugated to the solid phase always following the experimental procedure described by Hosoda [5], activating the carboxy groups of the spacer arm. The washing steps have been carried out through the use of a filter system in order to reduce the washing times. Then, a second amino acid has been bound to the first one by activating the α-carboxy groups of the first amino acid already immobilized. In this way, 144 dipeptides are obtained, after the final washes of the synthesis, 3 washes with a phosphate-citrate buffer (20 mM phosphate-citrate, 0.13 M NaCl, 1 mM EDTA, pH 4) are carried out for the purpose of changing the environment and the wells with the resin are stored in a dry state at 4°C until the time of use.

### Determination of the affinity and the bonding selectivity

0.25 ml/well of a ochratoxin A solution (2.0 µM) in a phosphate-citrate buffer are added at each of the 144 wells in which the resin is placed with the different sequences of the two immobilized amino acids. The reaction is proceeded for 24 hr at room temperature (RT). The non-specific bonding is evaluated by replacing the resin with the dipeptides immobilized with the resin coated with spacer arm. The total signal provided by the ochratoxin solution has been determined by distributing an equal volume of ochratoxin solution (2.0 µM) in four empty wells. The supernatant is recovered in an underlying plate by using the filter device. 100 µl for each well are transferred in the wells of a black plate, then the reading of the fluorometric signal is carried out. Moreover, a calibration curve of the ochratoxin is created. From these data and from the calibration curve the ochratoxin concentrations remained in solution, the non-specific signal and the total signal provided by the ochratoxin solution are obtained. The concentrations of bound ochratoxin can be then calculated from the experimental data obtained, and the ratio (B/F) concentration of bound ochratoxin (B) over concentration of free ochratoxin (F) has been used for expressing the affinity of each sequence towards the ochratoxin. Furthermore, the percentage selectivities have been calculated by dividing the B/F of a mycotoxin by the B/F of the ochratoxin and then multiplying by 100 the value obtained. The selectivity of the peptide selected for the ochratoxin was equal to 100%.

### Calculus of the affinity constants

The resin (0.5 g) with the selected peptide immobilized is suspended in 8 ml of phosphate-citrate buffer, 0.25 ml of suspension are distributed in the wells of a microplate with filter septa. The buffer is removed through filtration and 0.25 ml of an ochratoxin A solution at a different concentration are distributed. The concentrations of the toxin are distributed in duplicate and vary between (30 - 1.0 µM). The measurement is carried out twice for each dilution. In this case, the ochratoxin concentrations used have been rather high and for this reason this part of work has been carried out in a proper room of the department and using all the opportune security devices. The incubation is carried out for 24 hr at RT. The same dilutions are used for constructing a calibration curve which is distributed in the same plate where the resin is placed, and it is also left for a 24 hr incubation at RT. The supernatant is recovered in an underlying plate using the filter device. 100 µl for each well are transferred in the wells of a black plate, then the fluorometric reading is carried out. From the experimental data, the B/F ratios for each dilution and the concentration of bound ochratoxin can be calculated. By plotting these values, a Scatchard graph is created, from which the affinity constant and the bonding sites are obtained [6]. Always through the same equation it is possible, knowing the bonding sites and the B/F ratios of the other mycotoxins for the sequence under examination, to obtain the respective affinity constants.

### Creation of the second peptide library

After the selection of the dipeptide with the greater bonding abilities, the creation of a second peptide library is carried out. On the solid phase, the spacer arm and the sequence of the selected dipeptide have been immobilized following the same procedures described at the paragraph 2, then the immobilization of a third and a fourth amino acid is carried out by creating 144 tetrapeptides, where the first two amino acids are the same for every one and, on the contrary, the third and the fourth are different. The synthesis procedure is the same above described.

### Creation of the third peptide library

After the selection of the tetrapeptide with the greater bonding abilities, the creation of a third peptide library is carried out. On the solid phase, the spacer arm and the sequence of the selected tetrapeptide have been immobilized following the same procedures described at the paragraph 2, then the immobilization of a fifth and a sixth amino acid is carried out by creating 144 tetrapeptides, where the first four amino acids are the same for every one and, on the contrary, the fifth and the sixth are different. The synthesis procedure is the same above described.

### Evaluation of the experimental parameters on the hexapeptide - ochratoxin A bonding

It is known that there are some experimental parameters, such as the pH and the ionic strength, which can affect the affinity between the ligand and the analyte in question, therefore it has been decided to evaluate how the ochratoxin bonding to the hexapeptide varies (SEQ ID NO.: 1-8) selected from the library at nine different pHs and at five different ionic strengths, using proper buffers. The buffers used for the evaluation of the pH were phosphate/citrate buffers at the same ionic strength (0.13 M NaCl) with pH 2.2, 3, 4, 5, 6, 7, 8, respectively, in addition to two TRIS buffers (20 mM TRIS, 0.13 M NaCl) at pH 8 and 9; for the ionic strength, phospate/citrate buffers with the same pH (pH 4) with ionic strength 0, 0.1, 0.2, 0.5 respectively and 1 M (NaCl concentration) have been prepared.

### Evaluation of the bonding abilities of the hexapeptide towards the ochratoxin A at different pHs and at a different ionic strength

The resin suspended with the immobilized hexapeptide (SEQ ID NO.:1-8) has been distributed in 18 wells of a filter plate (0.25 ml/well); then, before distributing the ochratoxin solution on the resin, three washes each well with the buffers at a different pH in duplicate are carried out, so as to set the resin at the desired pH.

An ochratoxin A standard has been diluted in the different buffers at a different pH or a different ionic strength at the concentration of 2-10⁻⁶ M. Such solutions have been distributed (0.25 ml/well) in duplicate in the wells containing the resin and left to react for at least 24 hours at room temperature.

Furthermore, 0.25 ml of each buffer have been distributed in duplicate in a plate, in wells without resin.

The solutions contained in the wells are recovered the following day using the filter apparatus for plates; for each sample, the fluorescence reading at the fluorometer has been carried out (by collecting 100 µl of the solutions recovered from the plates) or in HPLC, using a Reverse Phase C-18 monolithic column, with a 1 ml/min flow rate and a 100 µl loop, using the eluants Q and R in a 44:56 ratio, with fluorescence detection (excitation λ 333 nm, emission λ 460 nm). The evaluation of the bonding abilities is carried out in the same way above described for the libraries.

### Adjustment of a small column containing the hexapeptide binding the OA by solid phase extraction

A quantity of resin containing the hexapeptide (SEQ ID NO.: 1-8) sufficient for filling a small column of about a 2 cm length and a 1 cm diameter, to be used for a step of solid phase extraction (clean-up) is synthesized, for the purpose of recovering the ochratoxin A by eliminating the interferences existing in the solution, in order to then carry out a quantitative evaluation of its concentration in HPLC with fluorescence detection.

For the synthesis of the resin, the procedure shown in the above paragraphs is followed; after having filled the small column with resin, washes with a MeOH:H₂O = 70:30 solution are carried out, for cleaning the resin from possible residues which could contaminate the following analyses (1 ml each time, leaving the solution into contact with the resin for at least 10 minutes).

Once such solutions have been discharged from the small column, three more washes are carried out with the buffer selected for the analyses (1 ml each time) and finally most of the buffer is eliminated, leaving only a few millimeters layer thereof, in order to avoid the resin from being dried.

For the adjustment of the solid phase extraction, different standard solutions of ochratoxin A in a phosphate-citrate buffer at pH 4, without addition of NaCl at different concentrations have been used, and the optimized extraction procedure is the following:
1. loading of 1 ml of ochratoxin A solution;
2. washing with 0.5 ml of phosphate-citrate buffer;
3. washing with 0.5 ml of acetonitrile;
4. elution with 1.5 ml of acetonitrile;
5. washing with 1 ml of phosphate-citrate buffer.

Fractions of 1 ml each have been collected and analyzed by HPLC using a Reverse Phase C-18 monolithic column, with a 1 ml/min flow rate and a 100 µl loop, using the eluants Q and R in a 44:56 ratio, always with fluorescence detection (excitation λ 333 nm, emission λ 460 nm).

In order to evaluate the ochratoxin A concentration recovered in the different fractions, a calibration line (5.0 x 10⁻¹¹ - 1.0 x 10⁻⁸ M) in HPLC has been constructed.

After each SPE it is convenient to carry out three regeneration washes with the MeOH:H₂O = 70:30 solution, for cleaning the resin from the ochratoxin A possibly remained in the column, which could contaminate the following analyses.

Once such solutions have been discharged from the small column, three more washes are carried out with the buffer selected for the analyses, in order to reequilibrate the small column, and finally most of the buffer is eliminated, only leaving a few millimeter layer thereof for avoiding the resin from being dried.

In order to evaluate the bonding ability of the peptide, the same kind of SPE and HPLC analyses is carried out using, instead of the resin with the immobilized hexapeptide, a resin simply blocked with aminobutyric acid, for showing the holding difference between a small column and the other one.

### Examination of the bonding abilities of the hexapeptide binding the ochratoxin A towards real matrixes

Once the SPE procedure has been optimized, the analysis of real samples and real samples additioned with ochratoxin A standards has been carried out; in particular, the additions carried out have been 1.10⁻⁸ M and 5.10⁻⁹ M, respectively corresponding with 4 ppb and 2 ppb. The SPE step, the collection of the fractions and the HPLC analysis are then carried out as described in the preceding paragraph.

The analyses have been carried out on the following wines:
- Roero Arneis of Montà d'Alba (CN - Piedmont);
- White wine, mixed grapes;
- Asti sparkling wine (Asti - Piedmont)
- Malvasia of Casorzo (AT - Piedmont);
- Merlot of Motta di Livenza (TV - Veneto);
- Marsala of Marsala (TP - Sicily);
- Piedmont Dolcetto of Casale Monferrato (TO - Piedmont);
- Home-produced French wine (Provence);
- Barbera of Asti (AT - Piedmont);
- Red wine, Piedmontese mixed grapes;
- Pelaverga (hills of Saluzzo - CN - Piedmont);
- Nebbiolo (Alba - CN - Piedmont);
- Dolcetto of Alba (Alba - CN - Piedmont);
- Rosato of Sicily;
- Cerasuolo of Montepulciano (Siena - Tuscany).

The analysis foresees the wine dilution with a 1% PEG 8000 solution, subsequent stirring, then filtration on a 0.45 µm filter. The solution is then loaded on the small column for the SPE extraction, and the mycotoxin is recovered following the optimized procedure in a buffer. A HPLC analysis is followed for the quantitative determination, as described in the preceding paragraph.

### EXAMPLE 1.

Having to individuate an amino acid sequence which shows evident molecular recognition properties towards the ochratoxin A', combinatorial libraries have been prepared.

The used amino acids have been selected so as to obtain a good chemical variety and different functional groups on the side chains; amongst the simplest amino acids, there are the glycine and the alanine, similar therebetween because of the small dimensions; then, there are the valine and the leucine, representative of the amino acids having hydrophobic chains, the tryptophan and the phenylalanine as aromatic amino acids, the lysine, the arginine and the histidine as hydrophilic amino acids, since having high polar side chains, the glutamine as a neutral amino acid, the serine for the hydroxyl group and the proline for the aliphatic side chain bound both to the α carbon atom and the nitrogen.

This amino acidic combination increases the chances of finding a suitable sequence, a chance which is also increased by the presence of the spacer arm, whose task is to space the peptide from the resin and make it more available to the interaction with the toxin.

The aminobutyric acid, used as a spacer arm, has been selected since, having per sé an amino acidic form, can act as an intermediary between the carboxyl of the resin and the following amino acids: in this way, the possible steric hindrance caused by the large dimensions of the resin particles with respect to the peptides is reduced, and furthermore, the bond chances of the analytes are increased, since the spacer arm could be able to contribute to the bond.

The 90% covering of the resin carboxy groups with ethanolamine, on the contrary, is useful for reducing the existing functional groups and having a more homogeneous distribution and the peptides more spaced therebetween, so as to allow a better bond with the toxins.

The dipeptide library has pointed out bonding abilities towards the ochratoxin A, as it can be seen by the graph reported in figure A: the sequence showing a greater affinity for the OA is the one formed by serine and asparagine (B/F = 0.32).

Therefore, using a Scatchard graph, the affinity constant can be calculated: being the concentration of mean sites calculated for the 50 µM dipeptide and the B/F obtained for the dipeptide Ser-Asn 0.32, the affinity constant results to be 7.94·10³ M⁻¹.

It has to be underlined that the bond of the aminobutyric acid alone towards the OA results almost null (B/F = 0.03) and therefore it does not help in any way the peptide-toxin bond.

The bonding ability of the dipeptides has been evaluated using a buffer at pH 4, as the real samples to be considered are wines, whose pH is generally between 3 and 4 [7].

### EXAMPLE 2.

In case of the tetrapeptide library, the peptides-OA bond has been evaluated not only at pH 4 but also at pH 8, in order to evaluate if, by exceeding the pKₐ value of the OA (pKₐ = 7.1), it is possible to increase the peptide affinity towards the toxin. In case of the dipeptides, this test has not been carried out, as usually the dipeptide bond results rather low and studies for improving the bonding abilities of the peptides result more useful on longer sequences.

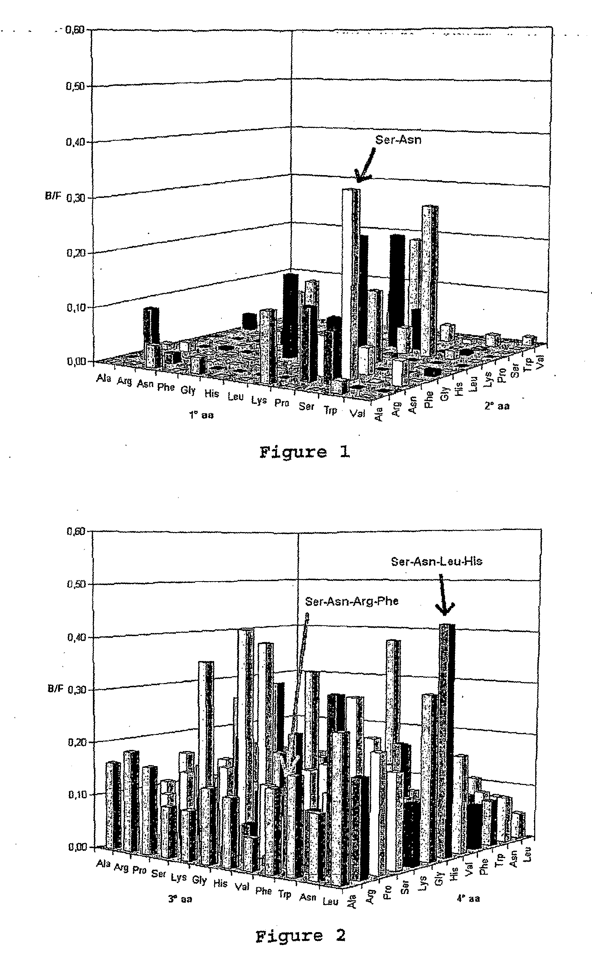

The tetrapeptide library has pointed out different bonding abilities towards the OA depending on the pH of the buffer used for setting the resin and for diluting the OA, as it can be seen from the graphs 2 and 3: generally, the bond results better at pH 4, in particular the sequence showing a greater affinity for the OA is the one formed by serine, asparagine, leucine and histidine (SEQ ID NO.: 9) at pH 4 (B/F = 0.43), whereas the same sequence at pH 8 has a much lower bonding ability (B/F = 0.09). At a pH 8, the serine, asparagine, arginine and phenylalanine sequence would result better, but having this latter a B/F only equal to 0.16 (and 0.14 at pH 4), the sequence ending with leucine-histidine is selected (figure 2).

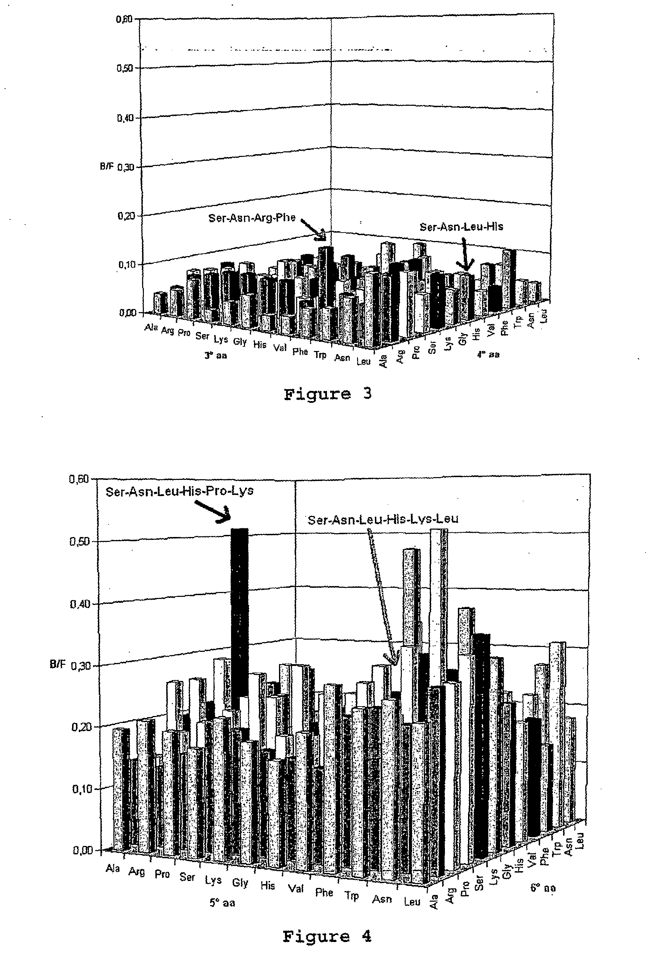

The affinity constant for the tetrapeptide towards the OA can be calculated always in the same way: being the concentration of mean sites calculated for the 30 µM tetrapeptide and the B/F obtained for the tetrapeptide Ser-Asn-Leu-His 0.43, the affinity constant is founds as 1.30·10⁴ M⁻¹ (figure 3).

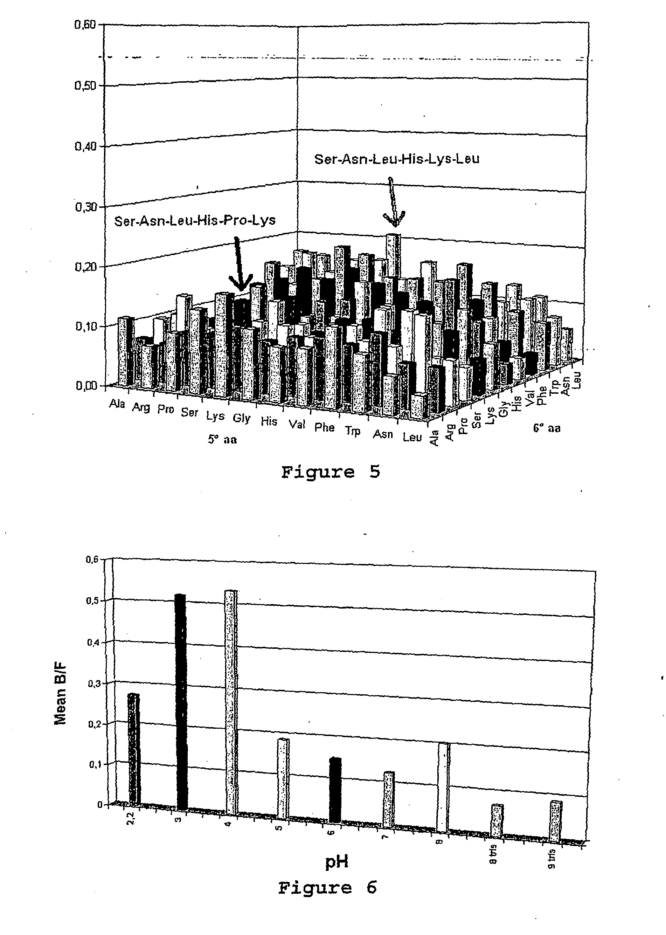

The hexapeptide library has pointed out bonding abilities towards the OA in a different way depending on the pH of the buffer used for setting the resin and for diluting the OA, as it can be seen from the figures 4 and 5: generally the bond, also in this case, results better at a pH 4, in particular the sequence showing a greater affinity for the OA is the one formed by serine, asparagine, leucine, histidine, proline and lysine (SEQ ID NO.: 1) at pH 4 (B/F = 0.52), whereas the same sequence at pH 8 has a bonding ability much lower (B/F = 0.12). At pH 8, the serine, asparagine, leucine, histidine, lysine and leucine sequence would result better, but having this latter a B/F only equal to 0.21 (and 0.21 at pH 4 as well), the sequence ending with proline-lysine is then selected (figure 4).

The affinity constant for the hexapeptide (SEQ ID NO.: 1-8) towards the OA (figure 5) can be calculated always in the same way: being the concentration of mean sites calculated for the 15 µM hexapeptide and the B/F obtained for the hexapeptide of SEQ ID NO.:1 0.52, the affinity constant is founds as 3.47·10⁴ M⁻¹.

This value of bonding constant can be considered satisfactory because it falls within the range of values (10⁴-10⁶ M⁻¹) which have proved to be sufficient for the application of the selected peptide to an affinity solid phase. Furthermore, during studies previously conducted, it has also be proven that after the six amino acids, the affinity constant does not increase anymore, but it stabilizes on the value obtained for the hexapeptide and that the selectivity towards molecules with a similar structure begins to decrease. For these reasons, the hexapeptide obtained has been subjected to additional studies in order to check its bonding features for the purpose of using the same for the preparation of a solid phase to be used in extraction procedures of the OA from wine samples.

The affinity constant obtained for the hexapeptide of SEQ ID NO.: 1 results to be in the order of magnitude of 10⁴, therefore it is advisable to carry out further studies on such sequence, for optimizing the experimental parameters, so as to try to increase such value.

So far, the peptide sequences were selected at pH 4 and pH 8 and ionic strength 0.13 M; in order to increase the affinity constant, it is tried to change such parameters, so as to define the optimal working conditions.

The first parameter taken into consideration was the pH, which has been changed from 2.2 to 9.

Bonding tests of the ochratoxin A at the same concentration but in different buffers (pHs varying from 2.2 to 9, ionic strength fixed at 0.13 M) were then carried out, in order to check which was the optimal pH.

From the bonding tests carried out (the B/F values are averages of duplicate values, with a mean standard deviation equal to 0.11 and CV% < 7.44), it has been possible to construct the graph reported is figure 6.

From the graph in figure 6, it is apparent that the reaction is greatly promoted at acid pHs, reaching higher B/F values at pH 3 and pH 4: having to carried out a selection between two very similar B/F values (respectively 0.52 and 0.53), the pH 4 is preferred, which is the one closer to the real pH of most of the wines, and furthermore between two high values, the selection of the higher is chosen as a strategy.

As the pKₐ of the ochratoxin is equal to 7.1, it is advisable to carry out an additional bonding test with HPLC detection in which, besides the buffer at pH 4, also a basic buffer at a pH greater than 7.1 is tested; amongst those analyzed, the basic buffer providing higher B/F valuers is the one at pH 8 (with a B/F mean value equal to 0.20).

After the ochratoxin solution has been left into contact with the resin containing the hexapeptide with the SEQ ID NO.: 1 overnight, the following day the solution is withdrawn and analyzed by HPLC.

The HPLC analysis has been carried out using a Reverse Phase C-18 monolithic column, with a 1 ml/min flux and a 100 µl loop, using the eluants Q and R in a 44:56 ratio [8], always with a fluorescence detection (excitation λ 333 nm, emission λ 460 nm).

Such test has confirmed the result previously obtained, as it can be noted by the graph reported in figure 6, which shows an average B/F of 0.54 at pH 4 and 0.14 at pH 8 (the B/F values are means of quadruple values, with a mean standard deviation equal to 0.08 and CV% < 6.51), confirming the selection of the acid pH for the following analyses.

The affinity constants obtained for the peptides of SEQ ID NO.:2-8 are reported in table 1.

**Table 1.**

| **Peptide** | **SEQ ID NO** | **kₐ (M⁻¹)** |
|---|---|---|
| Ser - Asn - Leu - His - Asn - Ser | 2 | 3,43·10⁴ |
| Ser - Asn - Leu - His - Phe - Gly | 3 | 3,20·10⁴ |
| Ser - Asn - Leu - His - Trp - Gly | 4 | 2,82·10⁴ |
| Ser - Asn - Leu - His - Asn - Gly | 5 | 2,58·10⁴ |
| Ser - Asn - Leu - His - Leu - Lys | 6 | 2,33·10⁴ |
| Ser - Asn - Leu - His - Trp - Ser | 7 | 2,22·10⁴ |
| Ser - Asn - Leu - His - Leu - Ser | 8 | 2.15·10⁴ |

### EXAMPLE 3

The second parameter taken into consideration was the ionic strength, which has been varied from 0 to 1 M, all at pH 4.

Bonding tests of the ochratoxin A at the same concentration were then carried out, but in different buffers (NaCl concentrations varying from 0 to 1 M, pH fixed at 4), in order to control which was the optimal ionic strength.

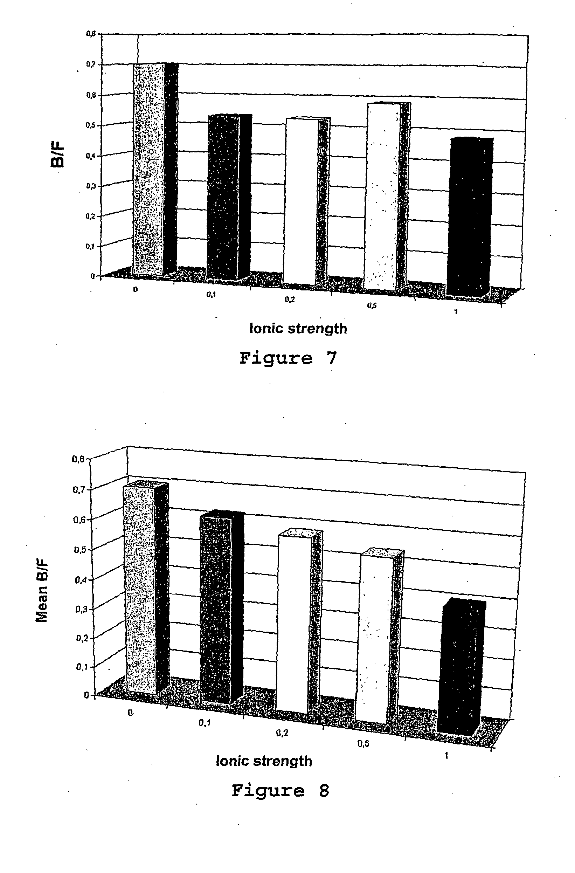

From the bonding tests carried out (the B/F values are means of doublets of values with a mean standard deviation equal to 0.15 and CV% < 1.26), it has been possible to construct the graph reported in figure 7.

The analysis of the obtained data shows how also the effect of the ionic strength remarkably affects the bonding abilities of the peptide of SEQ ID NO.: 1; from the results obtained, it is noted that a better B/F is obtained with a ionic strength equal to zero (B/F = 0.70, affinity constant = 4.67·10⁴ M⁻¹) . The optimal working conditions then result to be a buffer at pH 4 with a ionic strength equal to zero.

As for the evaluation of the best pH, it has been considered opportune to carry out a second control, and therefore the HPLC analysis has been carried out; the analysis conditions were the same used for the pH tests. After the ochratoxin solution has been left into contact with the resin containing the hexapeptide with the SEQ ID NO.: 1 overnight, the following day the solution is withdrawn and analyzed by HPLC.

Such test has confirmed the result obtained at the fluorometer, as it can be noted from the graph of figure 8: the B/F (the B/Fs are means of doublets of values with a mean standard deviation equal to 0.03 and CV% < 6.04) obtained at a ionic strength equal to zero continues to be the greatest amongst the five values found at the different ionic strengths.

In conclusion, for all the following analyses, a 20 mM phosphate/citrate buffer, 1 mM EDTA, pH 4, NaCl concentration = 0 M will be used.

### EXAMPLE 4.

For the purpose of checking the selectivity of the hexapeptide of SEQ ID NO.: 1 towards interfering molecules, but also for controlling if, possibly, it can be used also for the analyses of samples containing different toxins, it has been tested with respect to other mycotoxins: the mycotoxins taken into account were the aflatoxin B1, the B2, the G1 and the G2.

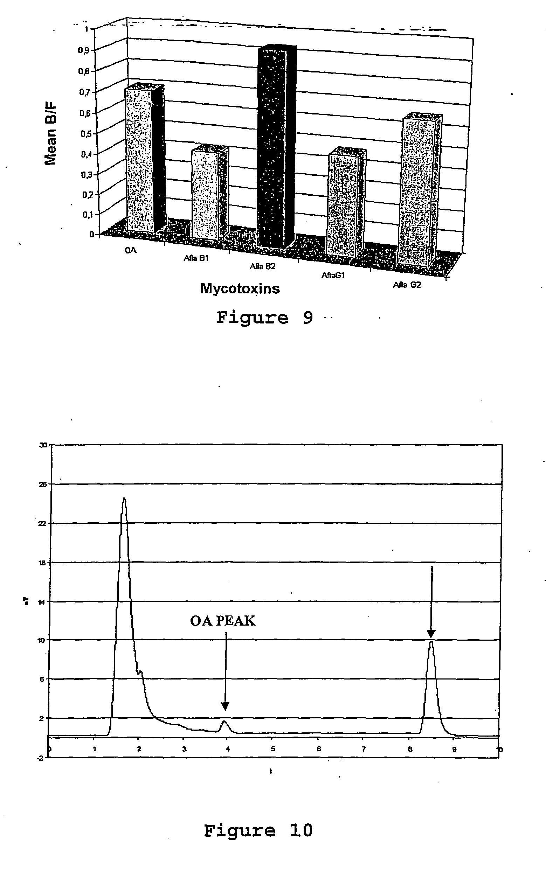

From the bonding tests carried out (the B/F values are means of values doublets, with a mean standard deviation equal to 0.01 and CV% < 3.86), it has been possible to construct the graph reported in figure 9.

From the B/F values obtained, it is possible to get the respective affinity constants:
- Ochratoxin A: 4.67·10⁴ M-¹;
- Aflatoxin B1: 2.87·10⁴ M⁻¹;
- Aflatoxin B2: 6.27·10⁴ M⁻¹;
- Aflatoxin G1: 3.2·10⁴ M⁻¹;
- Aflatoxin G2: 4.47·10⁴ M⁻¹.

It can be noted that the affinity constants are all around values of the order of magnitude of 10⁴ M⁻¹, with greater values for the ochratoxin A and the aflatoxin B2: therefore, this allows, in a theoretical way, to use the same peptide sequence also for clean-up steps of samples containing different mycotoxins (for example in the corn both types of toxins can be present), of which the presence and, in case, the HPLC concentration with fluorescence detection can be evaluated by using the wavelengths of maximum absorption of each of them; a further possibility is, after the SPE step, to simultaneously evaluate the presence of all the mycotoxins using a wavelength to which all of them absorb at least partly, by differentiating them with respect to the different retention time.

### EXAMPLE 5.

The Amberlite IRC-50 functionalized with the hexapeptide of SEQ ID NO.:1 has been used for a SPE small column to be used for carrying out the solid phase extraction for the purpose of recovering the ochratoxina A by eliminating the interferences existing in the solution, in order to then carry out a quantitative evaluation of its concentration in HPLC with fluorescence detection.

For quantitatively evaluating the concentration of ochratoxin A recovered, two calibration curves in HPLC have been constructed (respectively for evaluating higher and lower concentrations).

For the adjustment of the solid phase extraction, standard solutions of ochratoxin A in a phosphate-citrate buffer have been used. In the table 2, the extraction procedure optimized for the ochratoxin A standard in a phosphate-citrate buffer on a small column having the peptide immobilized and on a small column having the aminobutyric acid alone immobilized is reported.

**TABLE 2.**

| SPE fraction | Ochratoxin A column with peptide recovery (%) | Ochratoxin A "white" column recovery (%) |
|---|---|---|
| loading | 0 | 0 |
| 1^{st} wash with buffer | 0,17 | 11.38 |
| 1^{st} wash with MeCN | 0.24 | 50.58 |
| ELUTION (MeCN) | 101.11 | 37.86 |
| 2^{nd} wash with MeCN | 0.11 | 0.24 |
| 2^{nd} wash with buffer | 0 | 0 |
| *total recovery (%)* | *101.65* | *100.06* |

Once the SPE procedure has been optimized so as to recover the maximum possible of ochratoxin A in the second acetonitrile fraction, the analysis of real samples and added real samples of ocratoxin A standard has been carried out; in particular, the additions carried out were 1·10⁻⁸ M and 5·10⁻⁹ M, respectively corresponding with 4 ppb and 2 ppb, being this latter the legal limit for the ochratoxin A in the wines, determined by the European Commission with the Regulations 123/2005 dated 26/01/2005. The analysis has been carried out following a procedure described in literature [8] with the difference that, instead of the small immunoaffinity column, our small column with the immobilized peptide has been used, and the initial dilution step only foresees the use of a PEG solution and non PEG-hydrogen carbonate.

The results are reported in the following tables 3 and 4.

**TABLE 3.**

| **Sample** | | **OA (ppb)** | **Recovery (%)** |
|---|---|---|---|
| Red wines | malvasia | 4,00 | 91,4 |
| | | 2,00 | 75,1 |
| | merlot | 4,00 | 115,0 |
| | | 2,00 | 92,7 |
| | dolcetto | 4,00 | 115,8 |
| | | 2,00 | 91,6 |
| | dolcetto of alba | 4,00 | 99,4 |
| | doc | 2,00 | 92,8 |
| | French wine | 4,00 | 111,7 |
| | | 2,00 | 80,7 |
| | barbera | 4,00 | 109,3 |
| | | 2,00 | 117,0 |
| | Red mixed grapes | 4,00 | 101,8 |
| | piedmont | 2,00 | 102,5 |
| | pelaverga | 4,00 | 80,8 |
| | | 2,00 | 88,3 |
| | nebbiolo | 4,00 | 94,0 |
| | | 2,00 | 101,9 |

**TABLE 4.**

| **Sample** | | **OA (ppb)** | **Recovery (%)** |
|---|---|---|---|
| White wines | arneis | 4,00 | 84,8 |
| | | 2,00 | 99,0 |
| | | 2,00 | 112,8 |
| | Sparkling wine | 4, 00 | 122,9 |
| Rosé wines | Rosé of | 2,00 | 94,4 |
| | Sicily | 4,00 | 79,6 |
| | | 2,00 | 87,4 |
| | cerasuolo of Montepulciano | 4,00 | 82,5 |
| | | 2,00 | 86,3 |
| Dessert wines | marsala | 4,00 | 100,6 |
| | | 2,00 | 103,4 |

As it can be seen from the tables, the recoveries obtained are higher than 75% and completely compatible with those reported in literature for verified methods or which use, in the clean up step, small columns for immunoaffinity [9].

For a confirmation of the real ochratoxin measurement in the fraction eluted from the small column, this fraction has been subjected to confirmation for the presence of ochratoxin through the methylation method [10]: this method foresees the complete evaporation of the organic phase, therefore the ochratoxin is take up in methanol (500 µl) and concentrated hydrochloric acid (100 µl), stirred over a minute and left in the dark over 24 hours. In the chromatograms, the peak relating to the ochratoxin disappears and the peak relating to the methyl ester appears, confirming the original presence of ochratoxin.

By way of example, a chromatogram (figure 10) is reported, in which the displacement of the peak of the ochratoxin A at the greatest retention times can be observed.

In order to evaluate the reusability of the small column, the sample of Roero Arneis, additioned with 5·10⁻⁹ M of ochratoxin, has been repeated after 31 wine samples were passed on the same small column and the recovery percentage has resulted to be compatible with the one obtained the first time (105.2%). This result shows the concrete possibility of reusing many times the small column without loosing the bonding abilities of the hexapeptide, a thing that does not occur with the classical extraction systems by immunoaffinity.

In order to compare our procedure of solid phase extraction with the one carried out with a small column of classical immunoaffinity, we have analyzed a sample of white wine provided by ARPA of La Loggia (TO). This sample of white wine has been collected from a large bottle of white wine obtained by mixed grapes, the ochratoxin concentration found by ARPA was 1.57 ppb. Briefly, the method used by ARPA foresees the following procedure:
- dilution of the wine 1+1 with a 1% PEG aqueous solution, 5% NaHCO₃;
- solid phase extraction through the use of a commercial immunoaffinity small column;
- determination of the ochratoxin concentration via HPLC with fluorometric detector, mobile phase H₂O: MeCN:CH₃COOH=99:99:2 and commercial C18 column.

The same sample has therefore been analyzed using our procedure and attaining a concentration value equal to 2.22 ppb, a value completely consistent with the one provided from ARPA. Furthermore, a test of a standard addition has been made to ARPA sample with the addition of 2 ppb of an ochratoxin standard, and the recovery was 102.3%.

### EXAMPLE 6

For the preparation of stationary phases to be used in chromatography or in the solid phase extraction processes, it can be assumed the use of solid supports different from the crosslinked polystyrene (eg. Amberlite IRC-50), such as the agarose (ex. Sepharose), silica beads, latex particles and magnetic particles. The immobilization of the peptide takes place through the syntheses already described in the preceding paragraphs, by optionally changing the exposed functional group by means of passive coating of a suitable protein and/or covalent coating. This latter foresees the use of polyethylene glycols, trichlorotriazine and molecules with proper chemical properties (ex. aminobutyric acid, succinic acid, aminocaproic acid).

The peptide of interest is immobilized in wells of filter microplates for the purpose of obtaining a multichannel filter/sequestering system.

The peptide is fixed on the filter membrane using the same synthesis procedure shown for the SPE small column, when the membrane exposes carboxy groups. Furthermore, it can be assumed the use of a covering of the filter membrane, in case this latter does not offer adequate functional groups for the synthesis described in the preceding paragraphs, through the immobilization by covalent or passive coating of a protein (ex. BSA, succinilated BSA) or polylysines able to give a high number of functional groups for the immobilization reaction. Also the use of dendrimers or polyethylene glycols immobilized by covalent coating on the membrane on which the binding peptide is then grown must be taken into account.

The binding peptide can be exploited in the preparation of systems on strip or biosensors (ex. chips, micro- or nanosensors) for the field direct analysis of the ochratoxin A.

The peptides subject of the present invention can be applied to the development of systems for the removal of the ochratoxin A from the wine through the preparation of columns with an adequate quantity of resin and immobilized peptide. At present, on the resin Amberlite IRC-50 it results that only the 10% of the actually available functional groups is used.

Obviously, construction details and embodiments could be widely varied with respect to what has been described and shown, without leaving the protection ambit of the present invention, as it is defined by the appended claims.

### REFERENCES

1. Available at the address:
   http://www.lfra.co.uk/eman2/wp2analy.asp:
2. Tozzi C., Anfossi L., Giraudi G. J. Chromatogr. B 2003; 797: 289.
3. Tozzi C., Giraudi G. Curr. Pharm. Des. in press.
4. Tozzi C., Anfossi L., Giraudi G., Giovannoli C., Baggiani C., Vanni A., J. Chromatogr. A 2002; 966: 71.
5. Hosoda H., Sakai Y., Yoshida H. and Nambara H., Chem.Pharm.Bull. 27 (1971) 2147.
6. Tijssen P., in R.H. Burdon and P.H. van Knippenberg (Editors), Practice and theory of enzyme immunoassays, Elsevier, Amsterdam, The Netherlands, 1985, pp.126-130.
7. Internet site:
   pbi.wp.ebixtrade.it/site/pbi_wp_ebixtrade_it/NotaA plicativan1819.pdf
8. Visconti A., Pascale M., Centoze G., J.Chromatogr. A 864 (1999) 89.
9. Gilbert J., Anklam E.; Trac - Trends Anal. Chem. 21 (2002) 468.
10. Zimmerli B., .Dick R., J. Chromatogr. B 666(1995) 85.

## Claims

1. Binding system for mycotoxins including at least a peptide selected from the sequences SEQ ID NO.: 1-9.

2. Binding system according to claim 1, further including at least a spacer arm conjugated with said peptide.

3. Binding system according to claim 2, **characterized in that** said conjugation occurs through a covalent bond.

4. Binding system according to claim 2, **characterized in that** said spacer arm consists of aminobutyric acid.

5. Binding system according to any one of the claims 1 to 3, **characterized in that** said mycotoxin is ochratoxin A.

6. Use of a binding system according to any one of the claims 1 to 5, for the determination of ochratoxin A in a sample.

7. Use of a binding system according to any one of the claims 1 to 5, for the removal of ochratoxin A from a sample.

8. Use according to claim 6 or claim 7, **characterized in that** said sample is a food matrix, preferably wine.

9. Solid phase including at least a binding system according to any one of the claims 1 to 5 for the detection of ochratoxin A in a sample.

10. Solid phase according to claim 9, **characterized in that** said solid phase is selected from chromatography resin, microtitre plate, microtitre plate with filter septa, membrane, strip, sensor surface.

11. Process for the detection of ochratoxin A in a sample, including the following steps:
i) arranging a solid phase including at least a binding system according to any one of the claims 1 to 5;
ii) contacting said sample with said solid phase;
iii) detecting the bond between said binding system with ochratoxin A existing in said sample.

12. Process according to claim 10, **characterized in that** said solid phase is selected from chromatography resin, microtitre plate, microtitre plate with filter septa, membrane, strip, sensor surfaces.

13. Process according to claim 11, **characterized in that** said phase ii) is carried out under acid pH conditions.

14. Process according to claim 13, **characterized in that** said pH is between 2 and 5, preferably between 3 and 4.

15. Process according to any one of the claims 12 to 14, **characterized in that** said phase ii) is carried out under conditions with ionic strength lower than 0.5, preferably lower than 0.2.

16. Process for the removal of ochratoxin A from a matrix including the following steps:
i) arranging a solid phase including at least a binding system according to any one of the claims 1 to 5;
ii) contacting said matrix with said solid phase for a time sufficient for the formation of a bond between said binding system with ochratoxin A existing in said matrix;
iii) separating said matrix from said solid phase, so as to obtain a matrix free from ochratoxin A.

## Patentansprüche

1. Bindungssystem für Mycotoxine, einschließend mindestens ein Peptid, ausgewählt aus der Sequenz SEQ ID NR. 1-9

2. Bindungssystem nach Anspruch 1, ferner einschließend mindestens einen mit dem Peptid konjugierten Abstandhalterarm.

3. Bindungssystem nach Anspruch 2, **dadurch gekennzeichnet, dass** die Konjugation durch eine kovalente Bindung erfolgt.

4. Bindungssystem nach Anspruch 2, **dadurch gekennzeichnet, dass** der Abstandhalterarm aus Aminobuttersäure besteht.

5. Bindungssystem nach einem der Ansprüche 1-3, **dadurch gekennzeichnet, dass** es sich bei dem Mycotoxin um Ochratoxin A handelt.

6. Verwendung eines Bindungssystems nach einem der Ansprüche 1 bis 5 zur Bestimmung von Ochratoxin A in einer Probe.

7. Verwendung eines Bindungssystems nach einem der Ansprüche 1 bis 5 zur Entfernung von Ochratoxin A aus einer Probe.

8. Verwendung nach Anspruch 6 oder Anspruch 7, **dadurch gekennzeichnet, dass** es sich bei der Probe um eine Nahrungsmittelmatrix, vorzugsweise Wein handelt.

9. Feste Phase, einschließend mindestens ein Bindungssystem nach einem der Ansprüche 1 bis 5 zum Nachweis von Ochratoxin A in einer Probe.

10. Feste Phase nach Anspruch 9, **dadurch gekennzeichnet, dass** die feste Phase ausgewählt ist aus einem Chromatographieharz, einer Mikrotiterplatte, einer Mikrotiterplatte mit Filtersepten, einer Membran, einem Streifen, einer Sensoroberfläche.

11. Verfahren zum Nachweis von Ochratoxin A in einer Probe, einschließend die folgenden Schritte:
i) Anordnen einer festen Phase, einschließend ein Bindungssystem nach einem der Ansprüche 1 bis 5;
ii) Inkontaktbringen der Probe mit der festen Phase;
iii) Nachweisen der Bindung zwischen dem Bindungssystem mit in der Probe vorliegendem Ochratoxin A.

12. Verfahren nach Anspruch 10, **dadurch gekennzeichnet, dass** die feste Phase ausgewählt ist aus einem Chromatographieharz, einer Mikrotiterplatte, einer Mikrotiterplatte mit Filtersepten, einer Membran, einem Streifen, Sensoroberflächen.

13. Verfahren nach Anspruch 11, **dadurch gekennzeichnet, dass** die feste Phase ii) unter sauren pH-Bedingungen durchgeführt wird.

14. Verfahren nach Anspruch 13, **dadurch gekennzeichnet, dass** der pH-Wert zwischen 2 und 5, vorzugsweise zwischen 3 und 4 liegt.

15. Verfahren nach einem der Ansprüche 12 bis 14, **dadurch gekennzeichnet, dass** die feste Phase ii) unter Bedingungen mit einer Ionenstärke niedriger als 0,5, vorzugsweise niedriger als 0,2 durchgeführt wird.

16. Verfahren zur Entfernung von Ochratoxin A aus einer matrix, einschließend die folgenden Schritte:
i) Anordnen einer festen Phase, einschließend ein Bindungssystem nach einem der Ansprüche 1 bis 5;
ii) Inkontaktbringen der Probe mit der festen Phase für eine Zeitdauer, die zur Bildung einer Bindung zwischen dem Bindungssystem mit in der Probe vorliegendem Ochratoxin A ausreichend ist;
iii) Abtrennen der Matrix von der festen Phase derart, dass eine Matrix erhalten wird, die frei von Ochratoxin A ist.

## Revendications

1. Système liant pour mycotoxines incluant au moins un peptide choisi parmi les séquences SEQ ID n° : 1-9.

2. Système liant selon la revendication 1, comprenant en outre au moins un bras espaceur conjugué avec ledit peptide.

3. Système liant selon la revendication 2, **caractérisé en ce que** ladite conjugaison a lieu par l'intermédiaire d'une liaison covalente.

4. Système liant selon la revendication 2, **caractérisé en ce que** ledit bras espaceur est constitué d'acide aminobutyrique.

5. Système liant selon l'une des revendications 1 à 3, **caractérisé en ce que** ladite mycotoxine est l'ochratoxine A.

6. Utilisation d'un système liant selon l'une des revendications 1 à 5, pour la détermination de l'ochratoxine A dans un échantillon.

7. Utilisation d'un système liant selon l'une des revendications 1 à 5, pour l'élimination de l'ochratoxine A d'un échantillon.

8. Utilisation selon la revendication 6 ou la revendication 7, **caractérisé en ce que** ledit échantillon est une matrice alimentaire, de préférence du vin.

9. Phase solide incluant au moins un système liant selon l'une des revendications 1 à 5 pour la détection de l'ochratoxine A dans un échantillon.

10. Phase solide selon la revendication 9, **caractérisée en ce que** ladite phase solide est choisie parmi une résine de chromatographie, une plaque de microtitrage, une plaque de microtitrage avec septa de filtrage, une membrane, une bande, une surface de capteur.

11. Procédé pour la détection de l'ochratoxine A dans un échantillon, comprenant les étapes suivantes :
i) mise en place d'une phase solide incluant au moins un système liant selon l'une des revendications 1 à 5 ;
ii) mise en contact dudit échantillon avec ladite phase solide ;
iii) détection de la liaison entre ledit système liant avec l'ochratoxine A présente dans ledit échantillon.

12. Procédé selon la revendication 10, **caractérisé en ce que** ladite phase solide est choisie parmi une résine de chromatographie, une plaque de microtitrage, une plaque de microtitrage avec septa de filtrage, une membrane, une bande, une surface de capteur.

13. Procédé selon la revendication 11, **caractérisé en ce que** ladite phase ii) est exécutée dans des conditions de pH acide.

14. Procédé selon la revendication 13, **caractérisé en ce que** ledit pH est compris entre 2 et 5, de préférence entre 3 et 4.

15. Procédé selon l'une des revendications 12 à 14, **caractérisé en ce que** ladite phase ii) est exécutée dans des conditions de force ionique inférieure à 0,5, de préférence inférieure à 0,2.

16. Procédé d'élimination de l'ochratoxine A d'une matrice, comprenant les étapes suivantes :
i) mise en place d'une phase solide incluant au moins un système liant selon l'une des revendications 1 à 5 ;
ii) mise en contact de ladite matrice avec ladite phase solide pendant une durée suffisante pour que se forme une liaison entre ledit système liant et l'ochratoxine A présente dans ladite matrice ;
iii) séparation de ladite matrice d'avec ladite phase solide, de manière à obtenir une matrice dépourvue d'ochratoxine A.
